# EUROPEAN PATENT SPECIFICATION

(11) **EP 0 597 850 B1**
(45) Date of publication and mention of the grant of the patent: **02.10.1996**
(21) Application number: 91910825.8
(22) Date of filing: 13.06.1991
(51) Int. Cl.: C07K 14/00, C07K 1/00, A61K 9/22, A61K 38/00

(54) **CRYSTALLINE INTERLEUKIN-4**
KRISTALLINES INTERLEUKIN-4
INTERLEUKINE-4 CRISTALLINE

(30) Priority: 15.06.1990 US 538636
(43) Date of publication of application: 25.05.1994
(73) Proprietor: SCHERING CORPORATION, Kenilworth New Jersey 07033 (US)
(72) Inventor: HAMMOND, Gerald, East Orange, NJ 07018 (US); LE, Hung, V., Rockaway, NJ 07866 (US); NAGABHUSHAN, T., L., Parsippany, NJ 07054 (US); REICHERT, Paul, Montville, NJ 07045 (US); TROTTA, Paul, P., Secaucus, NJ 07094 (US)
(74) Representative: Ritter, Stephen David
(86) International application number: US9104045
(87) International publication number: WO9119742

(56) References cited:
- The Journal of Biological Chemistry, volume 262, no. 10, 5 April 1987, The American Society of Biological Chemists, Inc. (Baltimore, US), C. Sano et al.: "Crystallization and preliminary X-ray studies of human recombinant interleukin-2", pages 4766-4769, see page 4766, right-hand column, "crystallization"
- Journal of Molecular Biology, volume 218, no. 4, 20 April 1991, Academic Press Ltd (London, GB), W.J. Cook et al.: "Crystallization and preliminary X-ray investigation of recombinant human interleukin 4", pages 675-678, see the whole article

## Description

### BACKGROUND OF THE INVENTION

The T cell derived interleukin-4 (IL.4) was originally described as a murine factor that could co-stimulate the proliferation of activated B cells [Howard et al., J. Exp. Med. *155*:914 (1982)]. Subsequently, it was demonstrated that murine IL.4 could exert a variety of biological effects on B cells [Paul et al., Ann. Rev. Immunol. *5*:429 (1987); Noelle et al., Proc. Natl. Acad. Sci. USA *81*:6149 (1984); Roehm et al., J. Exp. Med. *160*:679 (1984); Vitetta et al., J. Exp. Med. *162*:1726 (1985); Coffman et al., J. Immunol. *136*:4538 (1986); Coffman et al., J. Immunol. *136*:949 (1986)] and other cell types including T cells [Mosmann et al., Proc. Natl. Acad. Sci. USA *83*:5654 (1986); Fernandez-Botran et al., J. Exp. Med. *164*:580 (1986); Hu-Li et al., J. Exp. Med. *165*:157 (1987); Grabstein et al., J. Immunol. *139*:1148 (1987); Zlotnick et al., Proc. Natl. Acad. Sci. USA *84*:3856 (1987)], hematopoietic progenitor cells [Rennick et al., Proc. Natl. Acad. Sci. USA *84*:6889 (1987); Peschel et al., Blood *70*:254 (1987)] and mast cells (Mosmann, *supra).*

Based on homology with murine IL.4, a cDNA encoding human interleukin-4 (hulL-4) has been cloned [Yokota et al., Proc. Natl. Acad. Sci. USA *83*:5894 (1986)] and expressed in both mammalian [Le et al., J. Biol. Chem. *263*:10817 (1988); Sonoda et al., J. Biotechnology 9:61 (1988); Takebe et al., Mol. Cell Biol. *8*:466 (1988)] and bacterial [van Kimmenade et al., Eur. J. Biochem. *173*:109 (1988)] hosts. Like murine IL.4, recombinant hulL-4 (rhulL-4) is a pleiotropic lymphokine that acts on a variety of cell types. Thus, for example, rhulL-4 can induce the proliferation of both activated T and B lymphocytes [Spits et al., J. Immunol. *139*:1142 (1987); DeFrance et al., J. Immunol. *139*:1135 (1987)], enhance the expression of class II major histocompatibility antigens and the low affinity receptor for IgE on B cells [Rousset et al., J. Immunol. *140*:2625 (1988); DeFrance et al., J. Exp. Med. *165*:1459 (1987)] and induce production of IgE and other immunoglobulins [Pene et al., Proc. Natl. Acad.Sci. USA *85*:6880 (1988)]. The ability of rhulL-4 to inhibit IL-2 dependent proliferation of chronic lymphocytic leukemic cells of B cell origin has suggested a clinical application in B cell neoplasms [Karray et al., J. Exp. Med. *168*:85 (1988)].

C. Sans et al. describe crystallization and preliminary x-ray studies of human recombinant interleukin-2.

The availability of milligram quantities of purified rhulL-4 has facilitated the initiation of studies of the structure and the structure-function relationships of the protein. The present invention relates to the discovery of conditions for producing crystalline rhulL-4. The invention further relates to the crystalline rhulL-4 itself. The crystals are suitable for X-ray diffraction studies and have applications in the purification and formulation of rhulL-4.

### SUMMARY OF THE INVENTION

In its broadest aspect the invention relates to Interleukin-4 in crystalline form, said crystalline form comprising tetragonal crystals of interleukin-4.

The invention further relates to human interleukin-4 in crystalline form.

The invention further relates to human interleukin-4 which has been produced by recombinant DNA techniques in crystalline form.

The invention further relates to interleukin-4 in crystalline form which may be glycosylated or non-glycosylated.

The invention further relates to a method for producing crystals of interleukin-4, which method comprises crystallizing interleukin-4 from a buffered pH 6-7 aqueous solution comprising a sulfate or citrate salt, with a concentration of interleukin-4 of 20 mg/ml.

### DESCRIPTION OF THE INVENTION

The crystalline interleukin-4 of this invention is useful for X-ray crystallographic analysis and for the preparation of pharmaceutical compositions for the treatment of any medical condition susceptible to treatment by interleukin-4.

To illustrate the practice of the present invention, the mature form of hulL-4 was expressed in *E. coli*. Other IL-4's as well as other forms of rhulL-4 may similarly be utilized. Purification of rhulL-4 from the cell supernatant was accomplished by conventional chromatographic methods (Le et al., *supra).* Hanging drop vapor diffusion experiments were performed in 24-well tissue culture plates (Multiwell, Becton Dickenson & Co, Lincoln Park, NJ). Droplets (6 µL) containing 20 mg/ml of hulL-4, 15-20% saturated ammonium sulfate, 40 mM sodium phosphate, pH 6.0-7.0, were hung from siliconized coverslips inverted into 24-well tissue culture plates. These droplets were equilibrated at either 12 or 22°C against 1 ml of 30-40% saturated ammonium sulfate, 40 mM sodium phosphate, pH 6.0-7.0. Controlled temperature incubation was performed in REVCO environmental chambers preset at 12° or 22°C.

The concentration of rhulL-4 in various buffers was determined spectrophotometrically using an extinction coefficient of 0.57/cm/mg/ml at 278 nm. Sodium dodecylsulfate polyacrylamide gel electrophoresis (SDS-PAGE) was performed according to the method of Laemmli [Nature *227*:680 (1970)]. Reversed phase HPLC was performed on a Rainin C4 column (4.6 x 250 nm; Dynamax, 300 Angstrom) developed with a linear gradient of acetonitrile in 0.1% trifluoroacetic acid. Determination of T cell proliferation activity using human peripheral blood lymphocytes was performed as described previously (Yokota et al., *supra).* For X-ray studies, large tetragonal crystals were mounted in glass capillaries and photographed with a precession camera at 22°C using CuKα radiation from a Rigaku RU-300 rotating anode. A complete native data set was collected on a Nicolet X-100A area detector using the same radiation source.

As previously stated, for purposes of illustration, the crystalline human interleukin-4 described in this invention is recombinant human interleukin-4 derived from *E*. *coli.* The primary structure of the protein is:

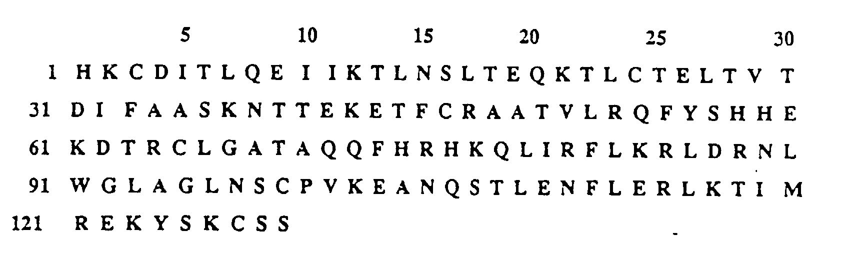

Using ammonium sulfate as precipitant rhulL-4 has been crystallized into needles and large tetragonal crystals. Crystallization may best be achieved in 40 mM sodium phosphate buffer, pH 6.0, containing 34% ammonium sulfate, after 21 days of incubation at 12°C. The tetragonal crystals have also been observed in sodium phosphate at pH values ranging from 5.0 to 7.0 and at concentrations of ammonium sulfate ranging from 30 to 40% saturation.

Crystallization is carried out at a pH range of 6 to 7, preferably at about pH 6.0. The temperature can range from about 5 to 25°C, preferably from 12 to 22°C and most preferably will be about 12°C. The protein concentration at equilibrium should be about mg/ml.

The period of incubation at 12°C to 22°C can vary from 2 to 20 days. It is anticipated that the crystallization methods described herein will also be useful for the crystallization of rhulL-4 derived from other host cells (e.g. mammalian cells in culture, yeast, insects and others) or hulL-4 derived from natural sources (e.g., human peripheral blood lymphocytes or human cell lines constitutively producing hulL-4). It is also anticipated that the method will be applicable to homologous interleukin 4 proteins derived from other species.

Crystalline rhulL-4 from the hanging droplets has been isolated and washed thoroughly in 55% ammonium sulfate, 40 mM sodium phosphate buffer, pH 6.6 at ambient temperature (22-25°C). After redissolution in 20 mM sodium phosphate buffer, pH 7.4, with 0.15 M sodium chloride, the crystals exhibited T cell proliferation activity (2.0 x 10⁷ units/mg) which was identical to that of a control rhulL-4 sample that had not been crystallized. When subjected to SDS-PAGE, the electrophoretic migration of the redissolved rhulL-4 crystals was identical to that of the control sample. Similarly, the reversed phase HPLC elution pattern of the redissolved crystals was indistinguishable from the elution pattern of the control sample. No protein degradation was apparent as a result of the long period of incubation at 22°C. More importantly, the process of crystallization followed by redissolution in physiological buffer failed to inactivate the T cell proliferation activity of rhulL-4.

X-ray diffraction data were initially collected to 2.7 Å resolution using the area detector. Oscillation frames covered 0.25 and were measured for 10 minutes. Indexing and integration of intensity data were carried out using the XENGEN processing programs [Howard et al., J. Appl. Crystallogr. *20*:383 (1987)]. The data indexed in the tetragonal system; with a = 92.1 (2), b = 92.1 (7) and c = 46.5 (1) Å. The space group is either P₄ 2₁ 2 or P4₃2₁2.

Subsequent X-ray precession photographs of interleukin-4 confirmed the space group and unit cell parameters. The tetragonal crystals are stable to x-rays and diffract at room temperature for at least five days and diffract to at least 2.7 Å resolution.

Large scale crystallization can be accomplished by methods equivalent to vapor diffusion, namely, dialysis and ultrafiltration. Seed crystals obtained from a hanging drop experiment can be used to accelerate the large scale crystallization once the optimum conditions have been established. Although the use of ammonium sulfate as a precipitant is preferred, it can be replaced by other common sulfate and citrate salts such as sodium, potassium, calcium or magnesium sulfate; sodium citrate, and others (McPherson, Preparation and Analysis of Protein Crystals, 1982, John Wiley & Sons, New York, New York). Large scale crystallization of rhulL-4 can be introduced as a final purification step and/or concentration step in clinical manufacturing. Long term storage of rhulL-4 bulk drug in a crystalline form is also highly desirable because of the inherent stability of the crystals as compared to rhulL-4 stored in solution with preservatives.

The crystals prepared by the described method also constitute a particularly advantageous form for pharmaceutical dosage form preparation. The crystals may be used, for example, as a basis for a slow-release formulation of rhulL-4 *in vivo.* It is believed that complexes of metals (e.g. zinc or iron) and hulL-4 could be formed and then subsequently crystallized. Crystals of these complexes could also be used in slow-release protein formulations containing appropriate pharmaceutical additives. Examples of similar slow-release protein formulations are the zinc-insulin crystalline complex (Remington's Pharmaceutical Sciences, 1985, Gennaro, A.R., Ed., Mack Publishing Co., Fasten, Pennsylvania, pp. 974-976) and the zinc-insulin-protamine crystalline complex (Pharmaceutical Manufacturing Encyclopedia, 1989, Sittig, M., Ed., pp. 820-821).

Slow-release pharmaceutical compositions comprising the crystalline interleukin-4 of the invention can be prepared by admixing such interleukin-4 with a metal and/or protein complexing agent as described above.

## Claims (Claims for the following Contracting State(s): AT, BE, CH, DE, DK, FR, GB, IT, LU, NL, SE)

1. Crystalline interleukin-4 comprising tetragonal crystals of interleukin-4.

2. The crystalline interleukin-4 of Claim 1 wherein the tetragonal crystals of interleukin-4 diffract x-rays to at least 2.7 Å.

3. The crystalline interleukin-4 of Claims 1 or 2 which is human interleukin-4.

4. The crystalline interleukin-4 of Claims 1 to 3 that is produced by recombinant DNA techniques.

5. The crystalline interleukin-4 of Claims 1 to 4 that is non-glycosylated.

6. The crystalline interleukin-4 of Claims 1 to 4 that is glycosylated.

7. The crystalline interleukin-4 of Claim 5 that is produced in E. coli.

8. A method for producing crystals of interleukin-4 which comprises incubating interleukin-4 in a buffered pH 6-7 aqueous solution comprising a sulfate or citrate salt and having a concentration of interleukin-4 at equilibrium of about 20 milligrams of interleukin-4 per millilitre of aqueous solution for a sufficient time to allow crystals of interleukin-4 to form.

9. The method of Claim 8 in which the buffered solution comprises ammonium sulfate at pH 6.0.

10. The method of any of Claims 8-11 further comprising incubating the IL-4 at a temperature of about 12°C.

11. A pharmaceutical composition comprising interleukin-4 in crystalline form as claimed in any one of Claims 1 to 7 and Claim 13 complexed with a metal and/or protein complexing agent.

12. A process for preparing a pharmaceutical composition of Claim 14 which comprises admixing interleukin-4 in crystalline form with a metal and/or protein complexing agent.

13. The use of interleukin-4 in crystalline form as claimed in any one of Claims 1-7 and Claim 13 for the preparation of a pharmaceutical composition for treating a medical condition susceptible to treatment by interleukin-4.

## Claims (Claims for the following Contracting State(s): ES)

1. A method for producing crystals of interleukin-4 which comprises incubating interleukin-4 in a buffered pH 6-7 aqueous solution comprising a sulfate or citrate salt and having a concentration of interleukin-4 at equilibrium of about 20 milligrams of interleukin-4 per millilitre of aqueous solution for a sufficient time to allow crystals of interleukin-4 to form.

2. The method of Claim 1 in which the buffered solution comprises ammonium sulfate at pH 6.0.

3. The method of any of Claims 1-4 further comprising incubating the IL-4 at a temperature of about 12°C.

4. A process for preparing a pharmaceutical composition which comprises admixing interleukin-4 in crystalline form, said crystalline form comprising tetragonal interleukin-4 crystals, with a metal and/or protein complexing agent.

## Claims (Claims for the following Contracting State(s): GR)

1. A method for producing crystals of interleukin-4 which comprises incubating interleukin-4 in a buffered pH 6-7 aqueous solution comprising a sulfate or citrate salt and having a concentration of interleukin-4 at equilibrium of about 20 milligrams of interleukin-4 per millilitre of aqueous solution for a sufficient time to allow crystals of interleukin-4 to form.

2. The method of Claim 1 in which the buffered solution comprises ammonium sulfate at pH 6.0.

3. The method of any of Claims 1-4 further comprising incubating the IL-4 at a temperature of about 12°C.

4. A process for preparing a pharmaceutical composition which comprises admixing interleukin-4 in crystalline form, said crystalline form comprising tetragonal interleukin-4 crystals, with a metal and/or protein complexing agent.

5. Use of crystalline interleukin-4 comprising tetragonal crystals of interleukin-4 in the manufacture of a medicament for treating a medical condition susceptible to treatment by interleukin-4.

## Patentansprüche (Patentansprüche für folgende(n) Vertragsstaat(en): AT, BE, CH, DE, DK, FR, GB, IT, LU, NL, SE)

1. Kristallines Interleukin-4, das tetragonale Kristalle von Interleukin-4 umfaßt.

2. Kristallines Interleukin-4 gemäß Anspruch 1, wobei die tetragonalen Kristalle von Interleukin-4 Röntgenstrahlen bis zu wenigstens 2,7 Å beugen.

3. Kristallines Interleukin-4 gemäß Anspruch 1 oder 2, bei dem es sich um Human-Interleukin-4 handelt.

4. Kristallines Interleukin-4 gemäß Ansprüchen 1 bis 3, das durch DNA-Rekombinationstechniken hergestellt wird.

5. Kristallines Interleukin-4 gemäß Ansprüchen 1 bis 4, das nicht glycosyliert ist.

6. Kristallines Interleukin-4 gemäß Ansprüchen 1 bis 4, das glycosyliert ist.

7. Kristallines Interleukin-4 gemäß Anspruch 5, das in *E*. *coli* gewonnen wird.

8. Verfahren zur Herstellung von Kristallen von Interleukin-4, umfassend das Inkubieren von Interleukin-4 in einer gepufferten wäßrigen Lösung von pH 6-7, die ein Sulfat-oder Citratsalz umfaßt und im Gleichgewicht eine Konzentration an Interleukin-4 von etwa 20 Milligramm Interleukin-4 pro Milliliter wäßriger Lösung besitzt, während einer Zeit, die ausreicht, daß sich Kristalle von Interleukin-4 bilden können.

9. Verfahren gemäß Anspruch 8, bei dem die gepufferte Lösung Ammoniumsulfat bei pH 6,0 umfaßt.

10. Verfahren gemäß Anspruch 8 oder 9, das weiterhin das Inkubieren des IL-4 bei einer Temperatur von etwa 12°C umfaßt.

11. Pharmazeutische Zusammensetzung, die Interleukin-4 inkristalliner Form gemäß einem der Ansprüche 1 bis 7 umfaßt, das mit einem Metall und/oder Proteinkomplexierungsmittel komplexiert ist.

12. Verfahren zur Herstellung einer pharmazeutischen Zusammensetzung gemäß Anspruch 11, das das Mischen von Interleukin-4 in kristalliner Form mit einem Metall und/oder Proteinkomplexierungsmittel umfaßt.

13. Verwendung von Interleukin-4 in kristalliner Form gemäß einem der Ansprüche 1 bis 7 zur Herstellung einer pharmazeutischen Zusammensetzung zur Behandlung eines medizinischen Zustands, der einer Behandlung mit Interleukin-4 zugänglich ist.

## Patentansprüche (Patentansprüche für folgende(n) Vertragsstaat(en): ES)

1. Verfahren zur Herstellung von Kristallen von Interleukin-4, umfassend das Inkubieren von Interleukin-4 in einer gepufferten wäßrigen Lösung von pH 6-7, die ein Sulfatoder Citratsalz umfaßt und im Gleichgewicht eine Konzentration an Interleukin-4 von etwa 20 Milligramm Interleukin-4 pro Milliliter wäßriger Lösung besitzt, während einer Zeit, die ausreicht, daß sich Kristalle von Interleukin-4 bilden können.

2. Verfahren gemäß Anspruch 1, bei dem die gepufferte Lösung Ammoniumsulfat bei pH 6,0 umfaßt.

3. Verfahren gemäß Anspruch 1 oder 2, das weiterhin das Inkubieren des IL-4 bei einer Temperatur von etwa 12°C umfaßt.

4. Verfahren zur Herstellung einer pharmazeutischen Zusammensetzung, das das Mischen von Interleukin-4 in kristalliner Form mit einem Metall und/oder Proteinkomplexierungsmittel umfaßt, wobei die kristalline Form tetragonale Interleukin-4-Kristalle umfaßt.

## Patentansprüche (Patentansprüche für folgende(n) Vertragsstaat(en): GR)

1. Verfahren zur Herstellung von Kristallen von Interleukin-4, umfassend das Inkubieren von Interleukin-4 in einer gepufferten wäßrigen Lösung von pH 6-7, die ein Sulfat-oder Citratsalz umfaßt und im Gleichgewicht eine Konzentration an Interleukin-4 von etwa 20 Milligramm Interleukin-4 pro Milliliter wäßriger Lösung besitzt, während einer Zeit, die ausreicht, daß sich Kristalle von Interleukin-4 bilden können.

2. Verfahren gemäß Anspruch 1, bei dem die gepufferte Lösung Ammoniumsulfat bei pH 6,0 umfaßt.

3. Verfahren gemäß Anspruch 1 oder 2, das weiterhin das Inkubieren des IL-4 bei einer Temperatur von etwa 12°C umfaßt.

4. Verfahren zur Herstellung einer pharmazeutischen Zusammensetzung, das das Mischen von Interleukin-4 in kristalliner Form mit einem Metall und/oder Proteinkomplexierungsmittel umfaßt, wobei die kristalline Form tetragonale Interleukin-4-Kristalle umfaßt.

5. Verwendung von kristallinem Interleukin-4, das tetragonale Kristalle von Interleukin-4 umfaßt, bei der Herstellung eines Medikaments zur Behandlung eines medizinischen Zustands, der einer Behandlung mit Interleukin-4 zugänglich ist.

## Revendications (Revendications pour l'(les) Etat(s) contractant(s) suivant(s): AT, BE, CH, DE, DK, FR, GB, IT, LU, NL, SE)

1. Interleukine-4 cristalline comprenant des cristaux tétragonaux d'interleukine-4.

2. Interleukine-4 cristalline de la revendication 1 où les cristaux tétragonaux d'interleukine-4 diffractent les rayons X jusqu'à au moins 2,7 Å.

3. Interleukine-4 cristalline des revendications 1 ou 2 qui est l'interleukine-4 humaine.

4. Interleukine-4 cristalline des revendications 1 à 3 qui est produite par des techniques d'ADN recombinant.

5. Interleukine-4 cristalline des revendications 1 à 4 qui est non glycosylée.

6. Interleukine-4 cristalline des revendications 1 à 4 qui est glycosylée.

7. Interleukine-4 cristalline de la revendication 5 qui est produite chez E. coli.

8. Méthode de production de cristaux d'interleukine-4 qui comprend l'incubation de l'interleukine-4 dans une solution aqueuse tamponnée à pH 6-7 comprenant un sel de sulfate ou de citrate et ayant une concentration d'interleukine-4 à l'équilibre d'environ 20 milligrammes d'interleukine-4 par millilitre de la solution aqueuse,pendant un temps suffisant pour permettre aux cristaux d'interleukine-4 de se former.

9. Méthode de la revendication 8, dans laquelle la solution tamponnée comprend du sulfate d'ammonium à pH 6,0.

10. Méthode selon l'une quelconque des revendications 8-11, comprenant de plus l'incubation de IL-4 à une température d'environ 12°C.

11. Composition pharmaceutique comprenant de l'interleukine-4 sous forme cristalline selon l'une quelconque des revendications 1 à 7 et la revendication 13 complexée à un agent complexant de métal et/ou de protéine.

12. Procédé de préparation d'une composition pharmaceutique de la revendication 14, qui consiste à mélanger l'interleukine-4 cristalline avec un agent complexant de métal et/ou de protéine.

13. Utilisation de l'interleukine-4 sous forme cristalline selon l'une quelconque des revendications 1-7 et la revendication 13 pour la préparation d'une composition pharmaceutique pour le traitement d'une condition médicale susceptible d'un traitement par l'interleukine-4.

## Revendications (Revendications pour l'(les) Etat(s) contractant(s) suivant(s): ES)

1. Méthode de production de cristaux d'interleukine-4 qui comprend l'incubation de l'interleukine-4 dans une solution aqueuse tamponnée à pH 6-7 comprenant un sel de sulfate ou de citrate et ayant une concentration d'interleukine-4 à l'équilibre d'environ 20 milligrammes d'interleukine-4 par millilitre de la solution aqueuse,pendant un temps suffisant pour permettre aux cristaux d'interleukine-4 de se former.

2. Méthode de la revendication 1, dans laquelle la solution tamponnée comprend du sulfate d'ammonium à pH 6,0.

3. Méthode selon l'une des revendications 1-4, comprenant de plus l'incubation de IL-4 à une température d'environ 12°C.

4. Procédé de préparation d'une composition pharmaceutique qui consiste à mélanger l'interleukine-4 sous forme cristalline, ladite forme cristalline comprenant des cristaux tétragonaux d'interleukine-4, avec un agent complexant de métal et/ou d'une protéine.

## Revendications (Revendications pour l'(les) Etat(s) contractant(s) suivant(s): GR)

1. Méthode de production de cristaux d'interleukine-4 qui comprend l'incubation de l'interleukine-4 dans une solution aqueuse tamponnée à pH 6-7 comprenant un sel de sulfate ou de citrate et ayant une concentration d'interleukine-4 à l'équilibre d'environ 20 milligrammes d'interleukine-4 par millilitre de la solution aqueuse, pendant un temps suffisant pour permettre aux cristaux d'interleukine-4 de se former.

2. Méthode de la revendication 1, dans laquelle la solution tamponnée comprend du sulfate d'ammonium à pH 6,0.

3. Méthode selon l'une des revendications 1-4, comprenant de plus l'incubation de IL-4 à une température d'environ 12°C.

4. Procédé de préparation d'une composition pharmaceutique qui consiste à mélanger l'interleukine-4 sous forme cristalline, ladite forme cristalline comprenant des cristaux tétragonaux d'interleukine-4, avec un agent complexant de métal et/ou d'une protéine.

5. Utilisation d'interleukine-4 cristalline comprenant des cristaux tétragonaux d'interleukine-4 pour la fabrication d'un médicament pour le traitement d'une condition médicale susceptible d'un traitement par l'interleukine-4.
